# EUROPEAN PATENT SPECIFICATION

(11) **EP 2 640 350 B1**
(45) Date of publication and mention of the grant of the patent: **26.09.2018**
(21) Application number: 11790712.1
(22) Date of filing: 18.11.2011
(51) Int. Cl.: A61K 8/42, A61K 8/64, A61Q 7/00, A61P 17/14, A61P 29/00, A61K 38/13, A61K 31/165

(54) **COMPOSITIONS AND METHODS FOR HAIR GROWTH**
ZUSAMMENSETZUNGEN UND VERFAHREN FÜR HAARWACHSTUM
COMPOSITIONS ET PROCÉDÉS POUR LA CROISSANCE DES CHEVEUX

(30) Priority: 18.11.2010 US 414937 P
(43) Date of publication of application: 25.09.2013
(73) Proprietor: Yoelin, Steven, Santa Ana, California 92701 (US)
(72) Inventor: Yoelin, Steven, Santa Ana, California 92701 (US)
(74) Representative: Hoffmann Eitle
(86) International application number: PCT/US2011/061489
(87) International publication number: WO 2012/068515

(56) References cited:
- US-A1- 2004 052 760
- US-A1- 2007 078 175
- US-B2- 7 351 404

## Description

### Field of the Invention

The present invention is directed to a composition for growing hair in a patent, wherein the composition comprises bimatoprost and cyclosporine, used concurrently and in combination. The present invention is also directed to compositions containing bimatoprost and cyclosporine to grow hair on the scalp for the treatment of all forms of hair loss not just alopecia areata see list below in green text of alopecia areata and various non-therapeutic methods of administration of the bimatoprost and cyclosporine compositions.

### Background of the invention:

Hypotrichosis or thinning hair of the scalp is a common medical problem that can often be socially debilitating. The mechanism of hair loss is unclear and is hypothesized to be caused by a variety of factors, including but not limited to hormonal imbalance, medications, infections and disease states. Current treatment options for hair loss can be lengthy with high rates of side effects or no effect at all, including steroid injection, minoxidil, and photochemotherapy. Oral cyclosporine A (CsA) has been shown to enhance hair growth in patients with moderate to severe alopecia, yet a high rate of discontinuation occurs due to systemic side-effects. Few studies have examined the efficacy and safety of topical cyclosporine A in animal models with hair loss. *In vivo* studies demonstrate that mice treated with 0.1% and 1% shift toward the anagen phase 21 weeks earlier than control mice, with significant hair growth observed after 2 weeks of treatment (Ezure, 2007). Topical 0.5% cyclosporine A applied BID to Experimental Bald Rats exhibited hair growth one week after application for 6 weeks with a reduction in follicular inflammation (Verma, 2004). Topical cyclosporine A has been shown to reduce dermal inflammation associated with atopic dermatitis (Saeki et al., 2009). The application of cyclosporine A as a topical to suppress T-cell function and enhance the anagen phase of the hair growth cycle is promising for patients with thinning hair.
In the foregoing passage, the article by Ezure can be found in the Journal of Dermatological Science 2007, vol. 47(2), pp.168-70; the article by Verma in the European Journal of Dermatology, 2004, vol 14(5), pp. 332-8; and the article by Saeki in The Journal of Dermatology, 2009, vol. 36, pp. 563-577. Later references to Ochoa and Uno can be found in the Journal of the American Academy of Dermatology, 2009, vol. 61(3), pp. 530-2 and the book "Hair and its disorders: biology, pathology and management", 2000, London: Martin Dunitz, pp. 137-151, respectively.
US 2004/052760 A1 describes a cosmetic composition containing at least one 15-hydroxyprostaglandin dehydrogenase inhibitor and cosmetically acceptable excipients for promoting the growth and/or preventing or delaying the loss of hair, as well as the use of a 15-hydroxyprostaglandin dehydrogenase inhibitor for the preparation of a composition intended for controlling hair loss and/or for promoting hair regrowth.
US 2007/078175 A1 describes phenylfurylmethylthiazolidine-2,4-dione and phenylthienylmethylthiazolidine-2,4-dione compounds that are reported as being useful for stimulating or inducing the growth of keratinous fibers and/or slowing down their loss and/or increasing their density and/or improving their appearance, and also for caring for or making up keratinous fibers, e.g., for caring for or making up the hair or eyelashes.

### Prostaglandins

Prostaglandins are a class of pharmacologically active hormone-like substances that mediate a wide range of physiological functions including blood pressure, smooth muscle contraction, inflammation and vascular permeability. A prostaglandin is any member of a group of lipid compounds that are derived enzymatically from fatty acids and have important functions in the animal body. Every prostaglandin contains 20 carbon atoms, including a 5-carbon ring. Prostaglandins are autocrine and paracrine lipid mediators that act upon platelets, endothelium, uterine and mast cells. They are synthesized in the cell from the essential fatty acids (EFAs). One example of an EFA is linoleic acid (LA). An LA derivative, gamma-linolenic acid (GLA) is known to reduce inflammation, and prostaglandins are known to regulate inflammatory mediation. The present invention recognizes that LA, GLA and prostaglandins may all play a role in hair health. In at least one embodiment of the present invention, the topical application of prostaglandins are used to enhance hair health and enhance hair growth.

There are nine types of prostaglandins, designated by the letters A to I, the degree of saturation of the side chain of each being designated by subscripts 1, 2, and 3. Examples of prostaglandins include prostaglandin E₁(alprostadil), prostaglandin E₂(dinoprostone), latanoprost and travoprost. Latanoprost and travoprost are actually prostaglandin prodrugs (i.e. 1-isopropyl esters of a prostaglandin) however, they are referred to as prostaglandins because they act on the prostaglandin F receptor, after being hydrolyzed to the 1-carboxylic acid.

A prostamide (also called a prostaglandin-ethanolamide) is a prostaglandin analog, which is pharmacologically unique from a prostaglandin (i.e. because prostamides act on a different cell receptor [the prostamide receptor] than do prostaglandins), and is a neutral lipid formed as a product of cyclo-oxygenase-2 ("COX-2") enzyme oxygenation of an endocannabinoid (such as anandamide). Additionally, prostamides do not hydrolyze in-situ to the 1-carboxylic acid. Examples of prostamides are bimatoprost (the synthetically made ethyl amide of 17-phenyl prostaglandin F_{2α}) and prostamide F_{2α}.

Bimatoprost, a prostaglandin analogue, has been shown to induce eyelash growth, resulting in increased length, thickness, and darkness in healthy patients (Latisse® PI, 2009). However, LATISSE® was found to have less effectiveness in promoting eyelash growth in patients with alopecia areata (Ochoa et al., 2009). This may be due to the inflamed tissue causing an inhibition or reduction in the ability of the drug to penetrate the follicle shaft, limiting its effectiveness. Uno et al. in 2001 reported significant hair growth in the bald scalp of macaques after 0.05% latanoprost. Density and thickness of hair increased significantly after 3 months of treatment. US Patent Nos. 6,403,649; 5,688.819; 5,474,979; and 4,839,342 are referred to.

### Immunomodulators

An immunomodulator, also known as an immunotherapy is a substance (e.g. a drug) which has an effect on the immune system. Immunomodulators may be used in compositions of the present invention, and include immunosuppressants, immunostimulants and tolerogens. Immunosuppressants inhibit immune response, for example, in autoimmune diseases. Immunostimulants increase the immune response, and can be useful, for example, in infections, immunodeficiency and cancers. Tolerogens induce tolerance and make tissue non-responsive to antigen.

Immunomodulators that can be used in compositions of the present invention include and are not limited to: cyclosporine, tacrolimus, azathioprine, cyclophosphamide, methotrexate, chlorambucil, mycophenolate mofetil, prednisolone, muromonab CD3, antithymocyte globin (ATG), Rho (D) immuneglobin, efalizumab, levamisole, thalidomide, and mixtures thereof. In at least one embodiment, the immunomodulator used in the composition is cyclosporine.

### Cyclosporine

As stated previously, the compositions of the present invention may contain cyclosporine or other active compounds. Cyclosporines are a group of nonpolar cyclic oligopeptides with known immunosuppressant activity. Cyclosporine A, along with several other minor metabolites, as well as cyclosporine B, C, D, E, F, G, H, I, J, K, L, M, N, O, P, Q, R, S, T, U, V, W, X, Y and Z, have been identified. In addition, derivatives, salts and the like of such cyclosporines and a number of synthetic analogs have been prepared and may be useful in the present invention. The use of cyclosporine-A and cyclosporine A derivatives to treat ophthalmic conditions has been the subject of various patents, for example Ding et al U.S. Patent 5,474,979; Garst U.S. Patent Nos. 6,254,860; 6,350,442, and 7,368,436.

In general, commercially available cyclosporines may contain a mixture of several individual cyclosporines which all share a cyclic peptide structure consisting of eleven amino acid residues with a total molecular weight of about 1,200, but with different substituents or configurations of some of the amino acids.

The term "cyclosporine component" as used herein is intended to include any individual member of the cyclosporine group, salts thereof, derivatives thereof, analogs thereof and mixtures thereof, as well as mixtures of two or more individual cyclosporines salts thereof and mixtures thereof. In certain embodiments, cyclosporine components include, without limitation, cyclosporine A, derivatives of cyclosporine A, salts of cyclosporine A and the like and mixtures thereof. Cyclosporine A is an especially useful cyclosporine component.

### Brief Description of the Drawings:

Figure 1 shows pictures of hair re-growth after 13 days of treatment with either vehicle, Cyclosporine (CsA) (0.05%, 0.1%, 1%) alone and in combination with Bimatoprost (0.03%, 0.3%, 3%) and Bimatoprost alone (0.03% and 0.3%); and,
Figure 2 shows Cyclosporine (CsA) alone (0.05%, 0.1%) and in combination with Bimatoprost (Bpst) (0.03%) stimulates hair-regrowth in shaved C57BL/6 male mice at day 13. The data for compositions comprising other than 0.03% w/v bimatoprost and 0.05% CSA do not form part of the invention and are provided for information purposes only.

### Summary of the Invention:

One aspect of the present invention is directed to the co-application of bimatoprost and cyclosporine A in the amounts specified in the claims to the scalp and other areas of the body to stimulate hair growth in normal patients (with or without hair loss). The invention also relates to compositions for growing hair in patients suffering from alopecia and other disorders resulting in hair loss including but not limited to androgen hormones including testosterone dihydrotestosterone, telogen, effluvium, poor nutrition, hair loss after surgery, hair loss after pregnancy, certain medications, hot oil treatments (perms etc.), excessive brushing, stress, traction alopecia, androgenetic alopecia, triangular alopecia, non-scarring alopecia, alopecia aerate, all other forms of alopecia.

The present invention is also directed to compositions and/or formulations containing both bimatoprost and cyclosporine A together in the amounts specified in the claims, to the scalp and other areas of the body to stimulate hair growth in normal patients (with or without hair loss) and in patients suffering from alopecia and other disorders resulting in hair loss including but not limited to androgen hormones including testosterone, dihydrotestosterone, telogen, effluvium, poor nutrition, hair loss after surgery, hair loss after pregnancy, certain medications, hot oil treatments (perms etc.), excessive brushing, stress, traction alopecia, androgenetic alopecia, triangular alopecia, non-scarring alopecia, alopecia aerate, all other forms of alopecia. The present invention is also directed to novel non-therapeutic methods of application of compositions containing cyclosporine A and bimatoprost in the claimed amounts to the scalp to grow hair or for treatment of hair loss.

Some embodiments of the invention are enclosed in the following items:
1) A composition for growing hair in a patient wherein the composition comprises 0.03% w/v bimatoprost and 0.05% w/v cyclosporine A.
2) The composition of item 1 wherein the composition further includes ethanol, propylene glycol, D-limonene and water.
3) The composition of item 2 wherein the composition further includes at least one excipient selected from the group consisting of Transcutol®, Labrasol®, cineole, Cremophor RH-40, DMSO, oleic acid, isopropyl myristate, propylene glycol, oxybutynin and monolaurate.
4) The compositions of item 3 wherein the composition is in the form of one selected from the group consisting of a liquid, suspension, emulsion, reverse emulsion, micro-emulsion, foam, semi-solid, solution, dispersion, capsule, gel, lotion, cream, paste, and polish.
5) The composition of item 2 wherein the composition is selected from the group consisting of a solution, foam, emulsion and gel.
6) The composition of item 5 wherein the composition is applied by an applicator.
7) The composition of item 3 wherein the composition consists essentially of 0.03% bimatoprost, 0.05% cyclosporine A, ethanol, propylene glycol, D-limonene and water.
8) The composition of item 7 wherein the composition further includes transcutol.
9) A composition comprising 0.03%) w/v bimatoprost and 0.05% w/v cyclosporine A, for use in a method of treating hair loss in a patient.
10) The composition of item 9 wherein the method includes applying a foam of 0.03% w/v bimatoprost and 0.05% w/v cyclosporine A at least once a day to the scalp.
11) The composition of item 10 wherein prior to application of the foam, the scalp is pretreated by application to the scalp of at least one of the following selected from the group consisting of heat, mechanical stimulation, sonophoresis, vibration and massage.
12) The composition of item 11 wherein the composition is applied on the scalp by application of a roll-on applicator.
13) A non-therapeutic method of growing hair on a human scalp comprising application of a composition comprising 0.03% w/v bimatoprost and 0.05 % w/v cyclosporine A.
14) The non-therapeutic method of item 13 wherein the composition is applied at least once a day.
15) The non-therapeutic method of item 13 wherein the composition is applied as one selected from the group consisting of a shampoo and conditioner.
16) The non-therapeutic method of item 13 wherein the composition is in the form of a liquid, suspension, emulsion, reverse emulsion, micro-emulsion, foam, semi-solid, solution, dispersion, capsule, gel, lotion, cream, paste, and polish.

### Detailed Description of the Invention:

### 1) Application of Latisse® and Restasis® alone in comparison to combined bimatoprost and cyclosporine A formulations:

One aspect of the present invention are combined compositions or formulations of cyclosporine ("CsA") and bimatoprost ("Bpst") on hair growth. Such formulations will be compared for their ability to stimulate hair growth to application of Latisse® and Restasis® applied individually to patients with thinning hair and with conditions resulting in hypertrichosis and other hair growth disorders.

### 1. Compositions containing both cyclosporine and bimatoprost

The present invention is directed in part to compositions or formulations containing both cyclosporine A, and bimatoprost in a single composition for use in growing hair and their non-therapeutic methods of application. The combination of cyclosporine A and bimatoprost is believed to be a safe and effective treatment in suppressing the inflammatory component of alopecia and other hair growth disorders h and in enhancing the amount of hair growth on the scalp in normal patients (patients without suffering from a hair loss disorder).
In accordance with the present invention, cyclosporine A is applied in the efficacious concentration of 0.05 % w/v together with bimatoprost in an amount of 0.03 % w/v in pharmaceutically acceptable carrier.

### a. Cyclosporine component

The compositions of the present invention comprise cyclosporine A.

Cyclosporines are a group of nonpolar cyclic oligopeptides with known immunosuppressant activity. Cyclosporine A, along with several other minor metabolites, as well as cyclosporine B, C, D, E, F, G, H, I, J, K, L, M, N, O, P, Q, R, S, T, U, V, W, X, Y and Z, have been identified. In addition, derivatives, salts and the like of such cyclosporines and a number of synthetic analogs have been prepared and may be useful in the present invention together with cyclosporine A. The use of cyclosporine A and cyclosporine A derivatives to treat various ophthalmic conditions, has been the subject of various patents, for example US Patent Nos. 5,474,979; 6,254,860; 6,350,442; and 7,368,436.

In general, commercially available cyclosporines may contain a mixture of several individual cyclosporines which all share a cyclic peptide structure consisting of eleven amino acid residues with a total molecular weight of about 1,200 Dalton, but with different substituents or configurations of some of the amino acids. Thus the present invention also contemplates mixtures of different types of cyclosporine or cyclosporine components together with cyclosporine A. The term "cyclosporine component" as used herein is intended to include any individual member of the cyclosporine group, salts thereof, and mixtures thereof, as well as mixtures of two or more individual cyclosporines salts thereof, and mixtures thereof.

Cyclosporine A is used in the compositions of the invention.

Further preferred cyclosporine components include derivatives of cyclosporine A, salts of cyclosporine A and the like and mixtures thereof.

The chemical structure for cyclosporine A is represented by Formula 1:

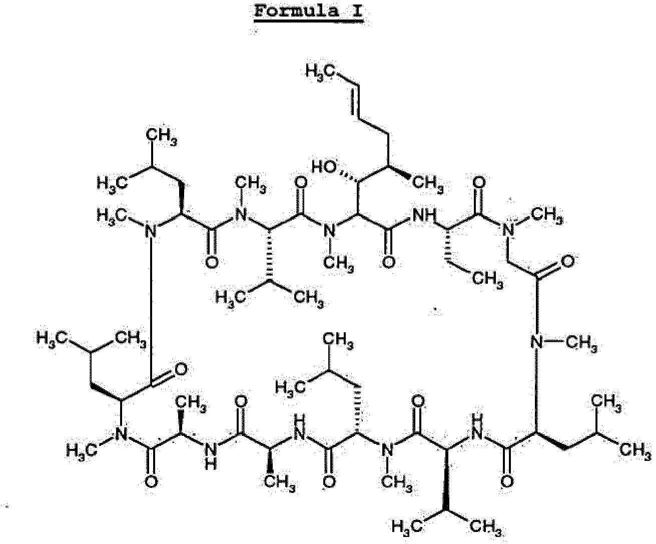

As used herein, the term "derivatives" of a cyclosporine refer to compounds having structures sufficiently similar to the cyclosporine.so as to function in a manner substantially similar to or substantially identical to the cyclosporine, for example, cyclosporine A, in the present compositions and non-therapeutic methods. Included within the useful cyclosporine A derivatives are those selected from ((R)-methylthio-Sar)³-(4'-hydroxy-MeLeu) cyclosporine A, ((R)-(Cyclo)alkylthio-Sar)³-(4'-hydroxy-MeLeu)⁴-cyclosporine A, and ((R)-(Cyclo)alkylthio-Sar)³-cyclosporine A derivatives described below.

These cyclosporine derivatives are represented by the following general formulas (II) and (III), respectively: 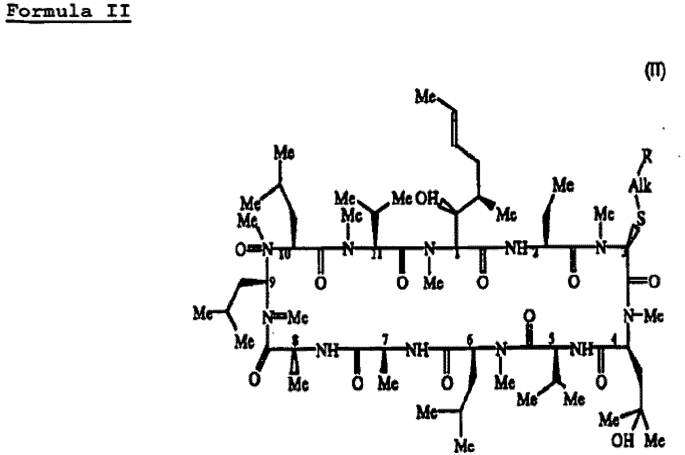 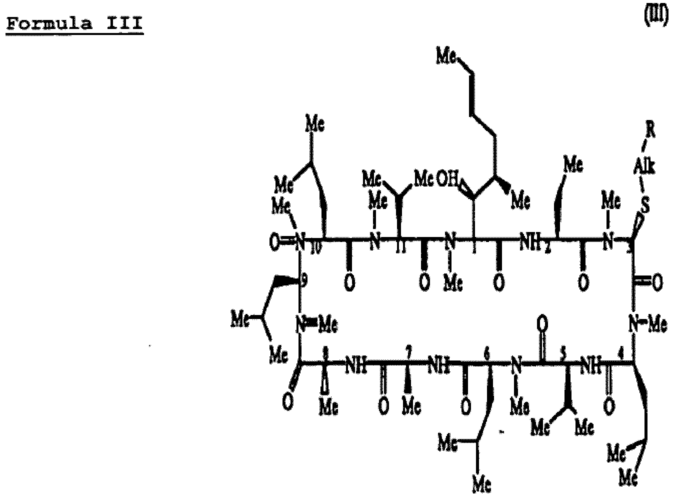 wherein Me is methyl; Alk is 2-6C alkylene or 3-6C cycloalkylene; R is OH, COOH, alkoxycarbonyl, -NR₁R₂ or N(R₃)-(CH₂)-NR₁R₂; wherein R₁, R₂ is H, alkyl, 3-6C cycloalkyl, phenyl (optionally substituted by halo, alkoxy, alkoxycarbonyl, amino, alkylamino or dialkylamino), benzyl or saturated or unsaturated heterocyclyl having 5 or 6 members and 1-3 heteroatoms; or NR₁R₂ is a 5 or 6 membered heterocycle which may contain a further N, O or S heteroatom and may be alkylated; R₃ is H or alkyl and n is 2-4; and the alkyl moieties contain 1-4C.

The present compositions and non-therapeutic methods may be practiced employing any suitable compositions or combinations of compositions including therapeutically effective amounts of cyclosporine component in conjunction with bimatoprost useful to promote hair growth. The cyclosporine component is present in an amount and/or concentration effective enough to provide the desired therapeutic effect when the cyclosporine-containing composition is administered to a human or animal in accordance with the present invention. Mixtures of cyclosporine components are contemplated. In the invention, the cyclosporine A is present in the compositions in an amount of 0.05% w/v of the composition.

### b. Bimatoprost

The present invention also involves the use of cyclosporine A in conjunction with bimatoprost, which may be represented generally by the formula I: 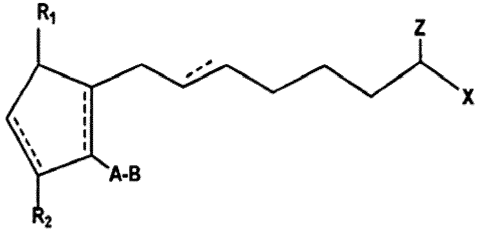 wherein the dashed bonds represent a single or double bond which can be in the cis or trans configuration, A is an alkylene or alkenylene radical having from two to six carbon atoms, which radical may be interrupted by one or more oxide radicals and substituted with one or more hydroxy, oxo, alkyloxy or akylcarboxy groups wherein said alkyl radical comprises from one to six carbon atoms; B is a cycloalkyl radical having from three to seven carbon atoms, or an aryl radical, selected from the group consisting of hydrocarbyl aryl and heteroaryl radicals having from four to ten carbon atoms wherein the heteroatom is selected from the group consisting of nitrogen, oxygen and sulfur atoms; X is a radical selected from the group consisting of -OR⁴ and -N(R⁴)₂ wherein R⁴ is independently selected from the group consisting of hydrogen, a lower alkyl radical having from one to six carbon atoms, R⁵-C- or R⁵-O-C-- wherein R⁵ is a lower alkyl radical having from one to six carbon atoms; Z is =O or represents 2 hydrogen radicals; one of R₁ and R₂ is =O, -OH or a -O(CO)R₆ group, and the other one is -OH or -O(CO)R₆, or R₁ is =O and R₂ is H, wherein R₆ is a saturated or unsaturated acyclic hydrocarbon group having from 1 to about 20 carbon atoms, or - (CH₂)mR₇ wherein m is 0 or an integer of from 1 to 10, and R₇ is cycloalkyl radical, having from three to seven carbon atoms, or a hydrocarbyl aryl or heteroaryl radical, as defined above, or a pharmaceutically-acceptable salt thereof, provided, however, that when B is not substituted with a pendant heteroatom-containing radical, and Z is =O, then X is not -OR⁴. (That is, the cycloalkyl or hydrocarbyl aryl or heteroaryl radical is not substituted with a pendant radical having an atom other than carbon or hydrogen.)

Bimatoprost may also be represented by the following general formula II: 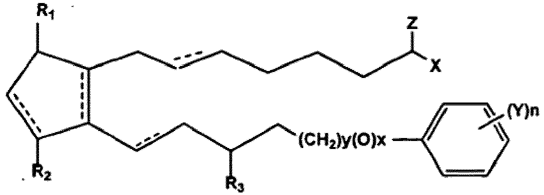 wherein y is 0 or 1, x is 0 or 1 and x and y are not both 1, Y is a radical selected from the group consisting of alkyl, halo, e.g. fluoro, chloro, etc., nitro, amino, thiol, hydroxy, alkyloxy, alkylcarboxy, halo substituted alkyl wherein said alkyl radical comprises from one to six carbon atoms, etc. and n is 0 or an integer of from 1 to about 3 and R₃ is =O, -OH or -O(CO)R₆ wherein R₆ is as defined above. Preferably, n is 1 or 2.

Bimatoprost may also be represented by the general formula (III). 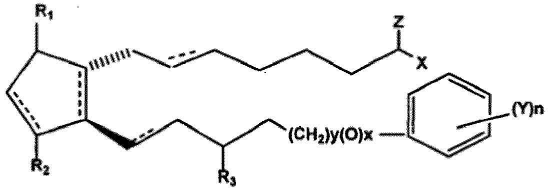 wherein hatched lines indicate a configuration, solid triangles are used to indicate □ configuration

Bimatoprost may also be represented by the general Formula (IV): 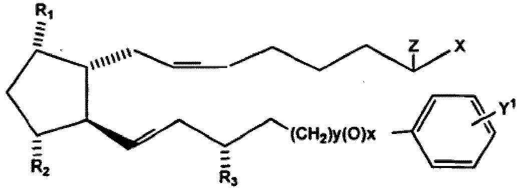 Or represented by the general Formula V:

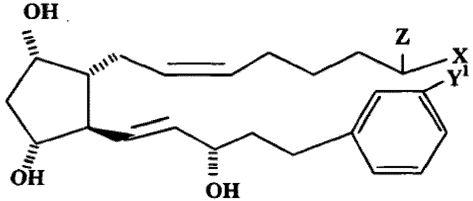

In all of the above formulae, as well as in those provided hereinafter, the dotted lines on bonds between carbons 5 and 6 (C-5), between carbons 13 and 14 (C-13), between carbons 8 and 12 (C-8), and between carbons 10 and 11 (C-10), indicate a single or a double bond which can be in the cis or trans configuration. If two solid lines are used that indicates a specific configuration for that double bond. Hatched lines at positions C-9, C-11 and C-15 indicate the α configuration. If one were to draw the β configuration, a solid triangular line would be used.

In the compounds used in accordance with the present invention, compounds having the C-9 or C-11 or C-15 substituents in the α or β configuration are contemplated. As hereinabove mentioned, in all formulas provided herein broken line attachments to the cyclopentane ring indicate substituents in the a configuration. Thickened solid line attachments to the cyclopentane ring indicate substituents in the α configuration. Also, the broken line attachment of the hydroxyl group or other substituent to the C-11 and C-15 carbon atoms signifies the α configuration.

For the purpose of this invention, unless further limited, the term "alkyl" refers to alkyl groups having from one to ten carbon atoms, the term "cycloalkyl" refers to cycloalkyl groups having from three to seven carbon atoms, the term "aryl" refers to aryl groups having from four to ten carbon atoms. The term "saturated or unsaturated acyclic hydrocarbon group" is used to refer to straight or branched chain, saturated or unsaturated hydrocarbon groups having from one to about 6, preferably one to about 4 carbon atoms. Such groups include alkyl, alkenyl and alkynyl groups of appropriate lengths, and preferably are alkyl, e.g. methyl, ethyl, propyl, butyl, pentyl, or hexyl, or an isomeric form thereof.

The definition of R₆ may include a cyclic component, -(CH₂)ₘR₇, wherein n is 0 or an integer of from 1 to 10, R₇ is an aliphatic ring from about 3 to about 7 carbon atoms, or an aromatic or heteroaromatic ring. The "aliphatic ring" may be saturated or unsaturated, and preferably is a saturated ring having 3-7 carbon atoms, inclusive. As an aromatic ring, R₇ preferably is phenyl, and the heteroaromatic rings have oxygen, nitrogen or sulfur as a heteroatom, i.e. R₇ may be thienyl, furanyl, pyridyl, etc. Preferably m is 0 or an integer of from 1 to 4.

Z is =O or represents two hydrogen atoms.

X may be selected from the group consisting of -OR⁴ and -N(R⁴)₂ wherein R⁴ is selected from the group consisting of hydrogen, a lower alkyl radical having from one to six 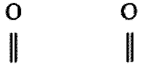 carbon atoms, R⁵-C- or R⁵-O-C- wherein R⁵ is a lower alkyl radical having from one to six carbon atoms.

Preferred representatives of the compounds within the scope of the present invention are the compounds of formula V wherein X is -OH, i.e. cyclopentane heptenoic acid, 5-cis-2-(3-α hydroxy-4-m-chlorophenoxy-1-trans-butenyl)-3,5-dihydroxy, [1_{α}, 2_{β}, 3_{α}, 5_{α}] and cyclopentane methylheptenoate-5-cis-2(3-αhydroxy4-m-chlorophenoxy-1-trans-butenyl)-3,5 dihydroxy, [1_{α},2_{β},3_{α},5_{α}] and the 9- and/or 11- and/or 15-esters of this compound. (The numbered designations in brackets refer to the positions on the cyclopentane ring.)

The following novel compounds may be used in the pharmaceutical compositions and the non-therapeutic methods of the present invention.
(1) cyclopentane heptenol-5-cis-2-(3α-hydroxy-5-phenyl-1-trans-pentenyl)-3,5-dihydroxy, [1_{α}, 2_{β}, 3_{α}, 5_{α}]
(2) cyclopentane heptenamide-5-cis-2-(3α-hydroxy-5-phenyl-1-trans-pentenyl)-3, 5-dihydroxy, [1_{α}, 2_{β}, 3_{α}, 5_{α}]
(3) cyclopentane N,N-dimethylheptenamide-5-cis-2-(3α-hydroxy-5-phenyl-1-trans-pentenyl)-3,5-dihydroxy, [1_{α}, 2_{β}, 3_{α}, 5_{α}]
(4) cyclopentane heptenyl methoxide-5-cis-2-(3α-hydroxy-5-phenyl-1-trans-pentenyl)-3,5-dihydroxy, [1_{α}, 2_{β}, 3_{α}, 5_{α}]
(5) cyclopentane heptenyl ethoxide-5-cis-2-(3α-hydroxy-4-meta-chlorophenoxy-1-trans-pentenyl)-3, 5-dihydroxy, [1_{α}, 2_{β}, 3_{α}, 5_{α}]
(6) cyclopentane heptenylamide-5-cis-2-(3α-hydroxy-4-meta-chlorophenoxy-1-trans-pentenyl)-3, 5-dihydroxy, [1_{α}, 2_{β}, 3_{α}, 5_{α}]
(7) cyclopentane heptenylamide-5-cis-2-(3α-hydroxy-4-trifluoromethylphenoxy-1-trans-pentenyt)-3,5-dihydroxy, [1_{α}, 2_{β}, 3_{α}, 5_{α}]
(8) cyclopentane N-isopropyl heptenamide-5-cis-2-(3α-hydroxy-5-phenyl-1-trans-penteny))-3,5-dihydroxy, [1_{α}, 2_{β}, 3_{α}, 5_{α}]
(9) cyclopentane N-ethyl heptenamide-5-cis-2-(3α-hydroxy-5-phenyl-1-trans-pentenyl)-3,5 dihydroxy, [1_{α}, 2_{β}, 3_{α}, 5_{α}]
(10) cyclopentane N-methyl heptenamide-5-cis-2-(3α-hydroxy-5-phenyl-1-trans-pentenyl)-3, 5-dihydroxy, [1_{α}, 2_{β}, 3_{α}, 5_{α}]
(11) cyclopentane heptenol-5-cis-2-(3α-hydroxy-4-meta-chlorophenoxy-1-trans-butenyl)-3,5-dihydroxy, [1_{α}, 2_{β}, 3_{α}, 5_{α}]
(12) cyclopentane heptenamide-5-cis-2-(3α-hydroxy-4-meta-chlorophenoxy-1-trans-butenyl)-3,5-dihydroxy, [1_{α}, 2_{β}, 3_{α}, 5_{α}]
(13) cyclopentane heptenol-5-cis-2-(3α-hydroxy-5-phenyl-1-trans-pentenyl)3,5-dihydroxy, [1_{α}, 2_{β}, 3_{α}, 5_{α}]

A pharmaceutically acceptable salt is any salt which retains the activity of the parent compound and does not impart any deleterious or undesirable effect on the subject to whom it is administered and in the context in which it is administered. Such salts are those formed with pharmaceutically acceptable cations, e.g., alkali metals, alkali earth metals, etc.

In one embodiment, the bimatoprost component is present in an amount of 0.03% w/v.

The listed concentration of cyclosporine A is combined with the listed concentration of bimatoprost in a composition having 0.05% w/v cyclosporine and 0.03 % w/v bimatoprost combined with the excipients and vehicles listed herein, such as, but not limited to, the vehicle in Example 1.

Useful compositions or formulations for practicing the invention may be in the form of liquids, suspensions, emulsions, reverse emulsions, micro-emulsions, semi-solids, solutions, dispersions, capsules, gels, lotions, creams, patch, foams, spray, pastes, polishes and the like. Those skilled in the art of pharmaceutical formulation are able to formulate suitable compositions including cyclosporine and bimatoprost components in a suitable form, such as those forms noted herein, for example, including one or more pharmaceutically acceptable excipients, such as those conventionally used in similar compositions.

### C. Excipients

Specific examples of pharmaceutically acceptable excipients included in the compositions of the present invention may be olive oil, arachis oil, castor oil, mineral oil, petroleum jelly, dimethyl sulphoxide, chremophor, Miglyol 182 (commercially available from Dynamit Nobel Kay-Fries Chemical Company, Mont Vale, N.J.), an alcohol (e.g. ethanol, n-propyl alcohol, or iso-propyl alcohol), liposomes or liposome-like products or a silicone fluid. Preferred excipients are dimethyl sulphoxide and olive oil. Mixtures of any suitable excipients are contemplated.

Excipients combinations which may be included in the compositions of the present invention, include, but are not limited to ethanol, propylene glycol: water (60:20:20). Other possible excipient combinations may include a ETOH: lipid: and water combination. Various penetrants/penetration enhancers may be used including but not limited to D-limonene, Transcutol®, Labrasol®, cineole, Cremophor RH-40, DMSO, oleic acid, isopropyl myristate, propylene glycol, oxybutynin and monolaurate. Botanicals including but not limited to Aloe Vera (this may also be used as a pre-treatment product)
http://www.scribd.com/doc/19139862/Aloe-VeraMiracle-Plant -Free Extract "Aloe Vera - The Magical Plant Amongst Us" by Dr. Reuben Titus.

The compositions may be in the form of micro emulsions (water, oil and surfactants) with various surfactants including but not limited to labrasol, transcutol, soy bean lecithin and cineole. Certain botanicals may also be used including but not limited to aloe vera which may be used as a pre-treatment product. Various Non-phosphorylated fatty acid oils may also be including but not limited emu oil and others listed on the internet website "www.hairloss-research.org" and various polyphenols including, but not limited to, epigallocatechin-3-gallate (EGCG) a major constituent of polyphenols (found in green tea). Accelerants may be included in the compositions including 8M-urea and dimethylsulphoxide (DMSO):
Various excipients, including but not limited to Ethanol: propylene glycol: water (60:20:20). Other possible excipients may include but are not limited to a ETOH: lipid: water combination. Grice et al. Relative Uptake of Minoxidil into Appendages and Stratum Corneum and Permeation through Human Skin In Vitro. J Pharm Sci 2010; Vol 99.
Various penetrants/penetration enhancers including but not limited to D-limonene. Fang et al. In Vitro and in vivo evaluations of the efficacy and safety of skin permeation enhancers using flurbiprofen as a model drug. Int J Pharmaceutics 2003; 255:153-166.
Various types of micro emulsions (water, oil and surfactants) including but not limited to Labrasol, Transcutol and cineole. Mura et al. Penetration enhancer-containing vesicles (PEVs) as carriers for cutaneous delivery of minoxidil. Int J Pharmaceutics 2009; 380: 72-79. Kreilgaard M et al. Influence of microemulsions of cutaneous drug delivery. Adv Drug Delivery Rev 2002; 1: S77-S98. Verma DD et al. Treatment of alopecia areata in the DEBR model using Cyclosporin A lipid vesicles. EJD 2004; 14:332-8.
Various Non-phosphoralated fatty acid oils including but not limited to Emu oil (see internet website www.hairloss-research.,org, Emu oil Hairloss and Frontal Regrowth, October 11th, 2010. Topical Emu Oil and Coconut Oil for Hair Loss- A Potent Combination July 15th, 2010.
Various Polyphenols including but not limited to epigallocatechin-3-gallate (EGCG) a major constituent of polyphenols (found in green tea), which can be used in a pretreatment solution as well (see internet web site www.hairloss-research.org Green Tea Consumption Grows Hair, Protects Against UV Radiation in Animal Models, June 24th, 2010.
Various nano-structure lipid carriers including but not limited to soybean lecithin. http://www.cosmeticsdesign.com/Formulation-Science/Nano-carriers-enhance-skin-penetration-and-antioxidant-effect-of-CoQ10Nano carriers enhance skin penetration and antioxidant effect of CoQ10, Katie Bird, 08-Apr-2010.
Various accelerants including but not limited to "8M-urea and dimethylsulphoxide (DMSO). MECHANISM OF ACTION OF ACCELERANTS ON SKIN PENETRATION ,.C. ALLENBY, N.H. CREASEN, J.A.G. EDGINTON, J.A. FLETCHER, and C. SCHOCK Article first published online: 29 JUL 2006 DOI: 10.1111/j.1365.2133. 1969.tb16061.x Issue British Journal of Dermatology Volume 81, pages 47-55, August 1969.
Various free base and acid addition salt forms. Skin penetration enhancement using free base and acid addition salt combination of active agents. US patent 4888354.

### D. Pretreatment Modalities

Various pretreatment modalities may be used to improve the efficacy of the present invention or increase penetration of the active compounds into the scalp. This includes varying the skin/scalp temperatures, including but not limited increasing the pre-treatment temperature of the area to be treated. Various forms of skin/scalp stimulation may also be employed including but not limited to mechanical stimulation, vibration and massage. Sound waves may also be utilized including but not limited to low frequency sonophoresis (ultrasound). This could be incorporated into the delivery device, for example a "roll-on" type of applicator could have a built in ultrasound device. Various forms of electrical stimulation including but not limited to sonophoresis/ultrasound and iontophoresis (for example Power Paper which is a iontophoresis type of product).

### E. Applicators

The compositions of the present invention may be applied to the treatment areas by various types of applicators including but not limited to spray pump bottles, aerosol bottles, and roll-on devices similar to "roll-on" antiperspirant applicators. The compositions may also be applied by shampoos or conditioners as well as other pretreatment products that would allow the actives that follow the shampooing/conditioning/pre-treating process to penetrate the scalp to the greatest degree possible. Also included are medicated shampoos that contain active pharmaceutical agents including but not limited to shampoos that contain ketoconazole. The shampoo known as Nizoral (ketoconazole) blocks the effects of DHT at the scalp, which may prevent hair loss. DHT is a testosterone metabolite that is believed to cause hair loss.

### Example I

Rapid Induction of Hair Growth by Penetration of Bimatoprost ("Bpst") and Cyclosporine A ("CsA") on Shaved Backs of Rodents.

### Objective

The objective of this non GLP study is to evaluate hair regrowth on male C57BL6 mice after single and combination therapy of cyclosporine A and bimatoprost every day for the first 20 days and ten every other day until Day 40 using a photography system. Documentation of observed skin pigmentation (correlating to hair follicle cycle) and skin irritation to detect dermal tolerability (dermatitis) every day for 40 days will also be evaluated. Mice weight will be recorded weekly to monitor health and appearance. Blood plasma will be collected for the pharmacokinetic determination of compounds via HPLC-MS for serum levels of interleukin-2 (IL-2) by ELISA. Tissue will be collected to determine hair follicle cycle (anagen, telogen, catagen) by histological evaluation at day 40.

### Dose Preparation

Test articles will be prepared once at the beginning of the study and will be prepared in a vehicle of 10% w/v EtOH, 2% w/v propylene glycol, 0.75% w/v carboxy methylcellulose, PBS (or ddw with pH adjustment). Cyclosporine A will be prepared at 0.05% (wt/v), 0.1% (wt/v), and 1% (wt/v) for groups 2, 4, 5, 7, and 8, respectively. Bimatoprost will be prepared at 0.03% (wt/v), 0.3% (wt/v), and 3% (wt/v), for dose groups 3, 4, 6, 7, and 8, respectively.

Based on stability information from the Sponsor, dose solutions will be prepared once at the beginning of the study and will be stored at 2-8 °C prior to administration. The bottles will be wrapped in aluminum foil to prevent exposing the test article to light.

### Test System and Husbandry

### General

| | |
|---|---|
| Species: | Mouse |
| Strain: | C57BL/6NCrIBR |
| Gender: | Male |
| Source: | Charles River |
| Initial Weight: | 20-30 g |
| Age: | 7 weeks |
| Number: | 120 |
| Identification: | Cage card and/or ear mark |
| Acclimation: | ≥ 7 days |

### Justification for Use of the Test System

Justification for the use of mice in this study is based on the premise that animal testing is an appropriate and ethical prerequisite to testing new drugs in humans, and that data obtained from nonclinical animal models will have relevance to the behavior of the test material in humans. Because of the complex interactions that occur *in vivo,* an *in vitro* system does not provide sufficient information for evaluation of a compound's *in vivo* activities (NIH, 1993). Mice have been used extensively to assess the nonclinical behavior (e.g., pharmacology, toxicity and pharmacokinetics) of a wide variety of pharmacological and toxicological (including environmental) agents. It is expected that the number of animals used in this study will provide a large enough sample for scientifically-meaningful results.

### Husbandry

*Housing and Enrichment.* Animals are maintained and monitored for good health in accordance with Pacific BioLabs animal husbandry SOPs. During acclimation, animals will be group housed in polycarbonate rodent boxes containing absorbent, bedding shavings. During study, animals will be individually housed in rodent boxes containing absorbent, bedding shavings.

*Acclimation Period.* Animals placed on study will have been acclimated to the testing facility for at least seven days prior to initiation of the study. Health observations will be performed periodically during acclimation to ensure acceptability for study; animals are placed on study at the discretion of the Study Director.

*Environment.* Animals will be maintained in a controlled environment with a temperature of 20 to 26°C, humidity of 50 ± 20%, and a light/dark cycle of 12 hours. The 12-hr lighting cycle may be interrupted to accommodate study procedures. The animals will be maintained in rooms with at least ten room air changes per hour. Vivarium facility records are kept on file at Pacific BioLabs.

*Diet and Feeding.* Animals will receive *ad libitum* certified (Laboratory Rodent Diet, etc), unless specified otherwise for dose administration. Analysis of food is provided by the manufacturer and representative reports of analyses are archived at Pacific BioLabs. There are no known contaminants in the dietary materials at levels expected to interfere with the conduct of this study.

*Drinking Water.* Fresh, potable drinking water will be available to all animals, ad libitum, via water bottle and sipper tubes. Water is supplied by the local utility and is analyzed two times per year by Pacific BioLabs for potential contaminants; results of water analyses are archived at Pacific BioLabs. There are no known contaminants in the water at levels expected to interfere with the conduct of this study.

*Identification.* Animals will be identified by cage cards and/or ear mark.

### Animal Welfare

*General Statement of Assurance.* This study will comply with all applicable sections of the Final Rules of the Animal Welfare Act regulations (9 CFR), the Public Health Service Policy on Humane Care and Use of Laboratory Animals, the Guide for the Care and Use of Laboratory Animals. The animals' living conditions, including housing, feeding, and non-medical care, will be appropriate for the species, contribute to their health and comfort, and will not deviate from the standards set forth in the USDA Animal Welfare Act and in the current Guide for the Care and Use of Laboratory Animals prepared by the Institute of Laboratory Animal Resources, National Academy of Sciences. Procedures used in this study have been reviewed and approved by the Pacific BioLabs Institutional Animal Care and Use Committee (IACUC) in compliance with the Animal Welfare Act [PBL ACUP 17-C09].

No animal will be used in more than one major operative procedure from which it is allowed to recover, unless scientifically justified or required as a veterinary procedure. Paralytics will not be used without appropriate anesthesia.
*Veterinary Care.* The purpose of the study is to establish the pharmacological and toxicological behavior of the test article, including adverse effects that may include pain or distress to the animal. The Study Director has reviewed the literature, and alternative tests that would avoid pain or distress are not currently available. Therefore, the withholding of palliative care, unless pain is severe (or chronic) or the animal is otherwise moribund, is justified.
Veterinary care will be available throughout the study, and animals found in severe distress may be treated to alleviate pain and suffering at the discretion of the consulting veterinarian. Such treatments will be noted in the study files and the Final Report, and the Sponsor will be notified of the additional veterinary care. Animals in severe distress or moribund may be euthanized at the discretion of the consulting veterinarian and the Study Director. The Sponsor will be consulted prior to euthanasia, if possible. Terminal procedures intended to euthanize animals will be conducted under the appropriate anesthesia as described by Pacific BioLabs SOPs.

Animals removed from the study may be replaced at the discretion of the Study Director, if replacement does not adversely affect study conduct.

### TEST PROCEDURES

### Experimental Design

Animals will receive daily topical application of the test article as summarized in Table 1. Doses will be administered to male C57BL/6 mice, with 12/mice group, and a total of 8 treatment groups for 40 days.

**Table 1. Group Designations and Dose Levels**

| Group # | Test Article | Gender | n | sDose Route | Dose Concentration or Amount (% w/v) | Dose Volume (ml/mouse/day) | Dose frequency |
|---|---|---|---|---|---|---|---|
| 1 * | Vehicle | M | 12 | Topical | 0 | 0.2 | single daily |
| 2 * | 0.05% CsA | M | 12 | Topical | 0.05% CsA | 0.2 | single daily |
| 3 * | 0.03% Bpst | M | 12 | Topical | 0.03% Bpst | 0.2 | single daily |
| 4 | 0.05% CsA 0.03% Bpst | M | 12 | Topical | 0.05% CsA 0.03% Bpst | 0.2 | single daily |
| 5 * | 0.1% CsA | M | 12 | Topical | 0.1% CsA | 0.2 | single daily |
| 6 * | 0.3% Bpst | M | 12 | Topical | 0.3% Bpst | 0.2 | single daily |
| 7 * | 0.1% CsA 0.3% Bpst | M | 12 | Topical | 0.1% CsA 0.3% Bpst | 0.2 | single daily |
| 8 * | 1% CsA 3% Bpst | M | 12 | topical | 1% CsA 3% Bpst | 0.2 | single daily |

| | | | | | | | |
|---|---|---|---|---|---|---|---|
| (*) These examples do not form part of the invention and are provided for information purposes only. | | | | | | | |

### Group Assignment

Animals will be assigned to treatment groups by the Study Director without apparent bias, but without randomization.

### Dose Administration

All animals will be shaved from the base of the ears to the base of the tail; care will be taken to avoid abrading the skin. The skin will be evaluated for pigmentation on the day prior to beginning treatment. All animals will be weighed weekly. Doses will be administered daily as a topical application.

### IN-LIFE OBSERVATIONS

### Body Weight Measurement

Body weights will be measured weekly (Day 1, 7, 14, 21, 28, 36, 40) and recorded.

### Moribundity/Mortality

General morbidity and mortality checks (e.g., cage-side observations) will be performed once daily.

### Clinical Observations

Clinical observations will be performed daily after dosing. Characteristics to be observed include general appearance of animal health and behavior.

### Other measures

Photos will be taken from the tip of the mouse nose to the base of the tail on a daily basis for the first 20 days of the study and then every other day until the conclusion of the study. Additionally, each mouse will be labeled (on cage card and tail) and each group will be color coded. Mice will be anesthetized with Isofluorane and the back skin of each mouse will be clipped and shaved carefully with electric clippers, so as not to damage the skin on study day 1. The hair removal will extend from the base of the ears to the base of the tail. Skin color will be assessed on a four point scale (P-1: pale pink (telogen), P-2: dark pink, P-3: light gray, P-4: dark grey/black (anagen)) and a photo will be taken after shaving. The skin should appear pale pink in color due to telogen phase of cycle (note: animals will not be used if skin is darker than pale pink). Photos will be downloaded and stored on computer and/or memory stick. Three rodents will be sacrificed for histological studies at Day 1 time point, prior to treatment application.

On day 39, 700 µl of blood will be collected from 6 animals from each treatment group by terminal cardiac puncture for pharmacokinetic analysis. 700 µl of blood will be dispensed into a green top tube containing sodium heparin and centrifuged at 2500g for 15 minutes to collect plasma. The plasma will be transferred to a microfuge tube and stored at -70 °C. An additional 700 µl of blood from three animals from each treatment group will be collected by cardiac puncture and transferred to serum separator tubes and centrifuged at 2500g for 15 minutes to collect serum for IL-2 analysis. The serum will be transferred to fresh tubes and stored at -70 °C until analysis.

On day 40, three mice/treatment group will be sacrificed by CO₂ asphyxiation and the skin will be excised from the treatment area and the skin will be fixed in 10% neutral buffered formalin.

**Table 2. Study Activities and Timetable**

| Day | Activity | Body Weights (Day) |
|---|---|---|
| 1 | Shave, Pictures, Score, Dose, Histology (3 rodents) | 1 |
| 1-20 | Score Pigmentation, Pictures, Dose | 7, 14 |
| 21-40 | Score Pigmentation, Pictures every other day, Dose | 21, 28, 36 |
| 39 | Cardiac Puncture (6 animals/group) for PK Cardiac Puncture (3 animals/group) for serum IL-2 | n/a |
| 40 | Histology tissue collection (3 animals/group) | 40 |

### TERMINAL OBSERVATIONS

### Post mortem Examinations

*Early Deaths.* Animals found dead will not be subject to a gross necropsy; organs and tissues from animals found dead will not be collected for histopathology.

*Scheduled Deaths.* Animals will not be subject to a gross necropsy at the scheduled termination; skin from the treatment area of these animals will be collected for histopathology.

*Euthanasia.* Animals will be euthanized with CO₂.

### Histopathology

The skin from the treatment area of three animals/group will be collected for subsequent histological evaluation on day 40 and fixed in 10% neutral buffered formalin.

### Collection Time Points

Pharmacokinetic samples will be obtained from study animals (6/group) on Day 39. Blood (700 µl) will be collected from animals by terminal cardiac puncture and transferred to potassium EDTA tubes, centrifuged for 15 minutes at 2500g, and plasma will be transferred to fresh tubes. Plasma samples will be stored at -70 °C and any analysis will be the responsibility of the sponsor.

Pharmacology samples for IL-2 analysis will be obtained from study animals (3/group) on Day 39. Blood (700 µl) will be collected from animals by terminal cardiac puncture and transferred to serum separator tubes, centrifuged at 2500g, and the serum will be transferred to fresh tubes and stored at - 60 to -80 °C. Analysis of the serum samples will be the responsibility of the sponsor.

### Collection and Processing

Blood samples will be collected from anesthetized animals via terminal cardiac puncture in an appropriately sized tube containing potassium EDTA as an anticoagulant for pharmacokinetic analysis or serum separator tubes for IL-2 analysis. Samples will be kept on wet ice until centrifugation at approximately 2800 rpm at room temperature for approximately 15 min. The plasma will be separated and stored at -60 to -80°C.

### Bioanalysis

Bioanalysis for test article plasma concentrations will be the responsibility of the Sponsor and bioanalytical results will not be included in the Final Report. Bioanalysis of IL-2 concentrations from serum will be the responsibility of the Sponsor and will not be included in the Final Report.

### DATA ACQUISITION AND ANALYSIS

Major computer software systems used on this study may include, but are not limited to, Microsoft Excel®, Microsoft Word®, and the Rees Scientific Environmental Monitoring System® for study room environmental control.

### Descriptive Statistics

Descriptive statistics (mean, standard deviations, and number of replicates) will be presented for all measurement data and will be shown in summary tables. Descriptive statistics will be calculated with Microsoft Excel®.

### Results

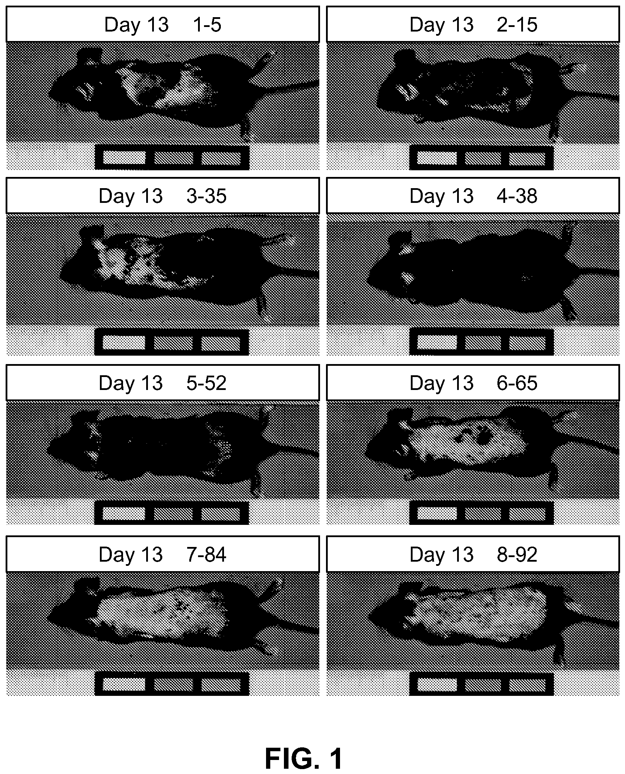

On Day 13, which is not the endpoint of the study (Day 40), hair growth of groups in Table 1 were evaluate as shown in Figure 1. As stated previously, 8 week old male C57BL/6 mice were shaved on the dorsal surface to remove the hair and the skin was scored for skin pigmentation as an indicator of telogen (pink) or anagen (dark gray). Only the mice with pink skin (telogen phase) on day one after shaving were included in the study. The mice were administered a daily topical application of 200 µl of either vehicle, CsA (0.05%, 0.1%, and 1%) alone and in combination with Bpst (0.03%, 0.3%, and 3%), or bimatoprost alone (0.03%, 0.3%) in a vehicle of 10% ethanol, 2% propylene glycol, 0.75% carboxymethylcellulose, and distilled de-ionized water with the pH adjusted to 7.4. On Day 13, the mice were anesthetized with Isofluorane and photos of the dosing area on each mouse were taken daily with a Nikon D90 digital camera mounted on a copy stand. From top left to right and down (group 1 animal 5, control), (group 2 animal 15, 0.05% CsA), (group 3 animal 35, 0.03% Bpst), (group 4 animal 38, 0.05% CsA/0.03% Bpst), (group 5 animal 52, 0.1% CsA), (group 6 animal 65, 0.3% Bpst), (group 7 animal 84, 0.1% CsA/0.3% Bpst), (group 8, animal 92, 1% CsA/3% Bpst). Groups 1-3 and 5-8 do not form part of the invention and are provided for information purposes only. These photos are shown in Figure 1.

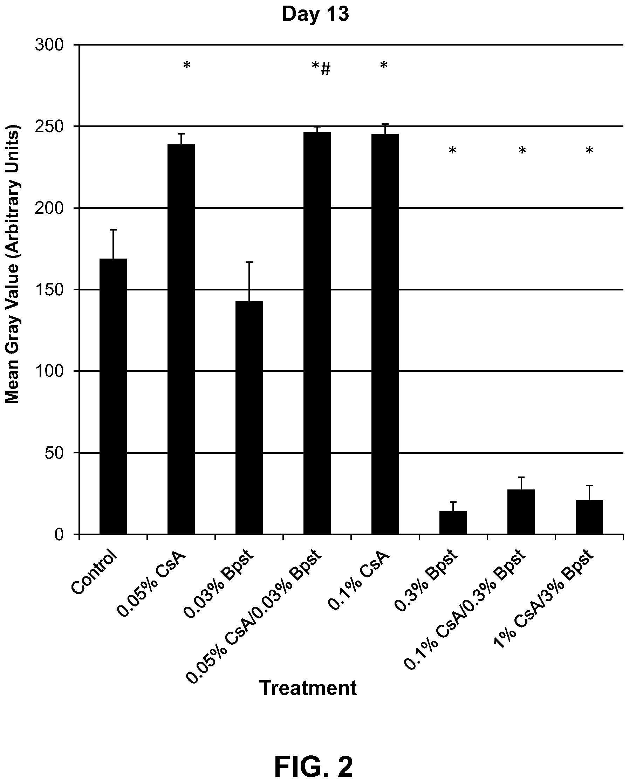

Figure 2 shows a rectangle (852x436 arbitrary units) was created using Image J software to encompass the shaved area on the dorsum of each mouse. The mean gray value of the rectangular area was measured using Image J software as an indicator of skin darkening and hair re-growth. indicates p<0.05 vs. control by t-test analysis using Microsoft Excel software. # indicates p<0.05 vs. 0.03% bimatoprost alone by t-test analysis using Microsoft Excel software. N=12/group ± S.E.M. The combination of 0.05% CsA and 0.03% Bpst demonstrated superiority over the control and 0.05% CsA treatment groups by stimulating skin darkening and hair re-growth by 46% over control treated mice and by 3% over 0.05% CsA alone. CsA alone at 0.05% and 0.1% do not form part of the invention and are provided for information only. These examples increased skin darkening and hair re-growth by 42% and 45% over controls, respectively. The combination treatment of 0.05% CsA and 0.03% bimatoprost increased skin darkening and hair re-growth by 0.4% over the 0.1% CsA alone treated group. The 0.05% CsA/0.03% Bpst group demonstrated superiority to 0.03% bimatoprost alone by stimulating skin darkening and hair re-growth by 73% over 0.03% bimatoprost alone at day 13 of the study.

### Example II

Purpose/Hypothesis: To demonstrate that the combination of cyclopsporine A ("CsA") and bimatoprost ("Bpst") are a safe and effective treatment in enhancing the length of hair growth on the scalp in patients with thinning hair and conditions resulting in hypotrichosis.

### Primary Outcome Measure:

To determine the penetration of single and combination therapy of cyclosporine A and bimatoprost for the treatment of hypotrichosis of the scalp in patients with thinning hair and conditions resulting in hypotrichosis.

### Secondary Outcome Measures:

Comparison of length, thickness, and amount of hair regrowth between the treatment groups:
1) To determine the change in growth of hair length at baseline and after application of single and combination therapy with cyclosporine A and bimatoprost;
2) To determine the safety of cyclosporine A by analysis of blood serum, specifically creatinine levels, thyroid hormone levels and immune cells, such as T-cell and interleukin counts, measured by laboratory testing;
3) To determine the safety of cyclosporine A by monitoring blood pressure measured by pressure cuff; and,
4) To determine the safety of bimatoprost by observation of changes in pigmentation of skin or dermatitis of the skin subjected to drug as measured by AOI software via Canfield photography.

Hair loss will be determined by questionnaire assessment and observation of scalp, where ≥ 1 square inch of the scalp must be thinning or without hair. The hair follicle must be determined to be active. On initial assessment, each patient will fill out a questionnaire, sign informed consent and have blood drawn. Selected patients will have photos taken of affected scalp area prior to treatment on Day 0.

Four groups of 10 patients will be assessed (total n=40). The four groups of patients will apply formulations (vehicle will have about 10% w/v ethanol, about 2% w/v propylene glycol, 0.75% w/v carboxymethylcellulose in distilled deionized water with the pH adjusted to 7.4 and further and may optionally including a penetration enhancer and/or preservative) of: 1) 0.05% CsA (Restasis®) alone (C0); 2) 0.03% Bpst (Latisse®) alone (B0); 3) a 0.05% CsA and 0.03% Bpst formulation (CB1); and 4) 0.1% CsA and 0.3% Bpst formulation (CB2). CO, BO, and CB2 do not form part of the invention and are provided for information purposes only. "About" refers to variations in concentration of actives or excipients that a regulatory agency such as the FDA or EMEA would find bioequivalent.

All patients will clean and dry their scalp in either the morning or evening, followed by a single treatment of product to treatment area of scalp 1 time/evening (qhs). Treatment should remain on the scalp for at least 8 hours. Patients will keep a diary with weekly entries regarding hair growth and any adverse events. The office visits will be conducted on the initial day of the trial, Day 0, at 2 weeks, followed by monthly visits for a duration of 6 months. A total of 8 visits will be required and at each visit, photographs of the treatment area will be taken with AOI software. This will be calculated by the percent of affected area per cm2 demonstrating growth, as measured by AOI software via Canfield photography. At Day 0 and Month 6, there will be assessment of blood pressure via pressure cuff and a blood draw followed by blood laboratory analysis, with attention to creatinine and thyroid levels, and immune cell count of T-cells and interleukins.

### Subject Characteristics (Inclusion criteria):

1) Subjects must give written informed consent and must authorize release and use of health information;
2) Male and female patients must have a diagnosis of hair loss or thinning hair with patches or over entire scalp as determined by the study investigator;
3) Male and female patients ≥ 18 years to 65 years of age; and,
4) Subjects must have CD4+ T-lymphocyte counts at lower limit of normal as determined by local laboratory.

### Subject Characteristics (Exclusion criteria):

1) Autoimmune disorders, immune suppression or evidence of immunocompromise;
2) Abnormal T-lymphocyte count and/or liver function tests or serum hemoglobin;
3) Pregnant or breastfeeding women;
4) Hypertension, taking hypertensive medication or unstable cardiovascular disease;
5) IOP lowering medications;
6) Currently taking steroid medications or steroid derivatives, or hormone supplements;
7) History of male pattern baldness ;
8) History of tricotillomania or patients with damaged hair follicles; and,
9) Other unspecified reasons that contraindicate enrollment in the study, as determined by the investigator

### Statistical Analysis Plan:

The primary and secondary outcome measures will first be determined using 3-way ANOVA, with patient, pretreatment (bimatoprost or placebo), and treatment (cyclosporine or placebo) as the between-subjects factors. Subsequent analyses of variance using 2-way ANOVA will be used to determine the effects of pretreatment and treatment within the patient group. If there are main effects of pretreatment or treatment, or an interaction effect, post hoc analyses using Bonferroni correction will be used to determine the source of these effects. A p-value of 0.05 is significant.

Results will demonstrate that combined formulations of cyclosporine and bimatoprost alone are superior in enhancing hair growth in patients with thinning hair and in patients suffering from hypertrichosis or other hair-loss disorders.

### Example III

A 47 year old Caucasian male suffering from alopecia areata applies a 0.03%/0.05% w/v bimatoprost/cyclosporine A solution to his scalp by applying the solution twice a day with an applicator, once in the morning and once at night for a period of 60 days. Hair growth is measured once a week by calculating the percent of affected area per cm2 demonstrating growth, as measured by AOI software, via Canfield photography. After 60 days of twice daily application of the 0.03%/0.05% w/v bimatoprost/cyclosporine A solution, a 27% increase in hair growth will be measured.

The following examples do not form part of the invention and are provided for information purposes only.

### Example IV

A 35 year old Caucasian female with thinning hair applies a 0.3%/0.5% w/v bimatoprost/cyclosporine A emulsion to her hair once a day with a roll-on applicator for a period of 90 days. Hair growth is measured weekly by calculating the percent of affected area per cm2 demonstrating growth, as measured by AOI software, via Canfield photography. After 90 days of daily application of the 0.3%/0.5% w/v bimatoprost/cyclosporine A solution, a 31% increase in hair growth and fullness will be measured.

### Example V

A 57 year old African American female with alopecia areata and thinning eyebrows applies a 0.1%/0.3% bimatoprost/cyclosporine A foam to her scalp and eyebrows twice a day, once in the morning and once at night, for a period of 90 days. The foam is applied and allowed to remain on the skin for 15 minutes after pretreatment of the skin with a warmed, moist towel to increase the temperature of the treatment area. After 90 days of application of the 0.1%/0.3% bimatoprost/cyclosporine A foam, a 26% increase in hair growth is observed on the scalp and an increase of 21% of eyebrow growth will be observed using AOI software and Canfield photography.

### Example VI

A 27 year old Hispanic male suffering from male pattern baldness and alopecia areata applies a 0.2%/0.4% w/v bimatoprost/cyclosporine A gel to his hair once daily for a period of 120 days. The gel is applied to the scalp and is allowed to dry. Hair growth is measured weekly by calculating the percent of affected area per cm2 demonstrating growth, as measured by AOI software, via Canfield photography. After 120 days of daily application of the 0.2%/0.4% w/v bimatoprost/cyclosporine A gel, a 24% increase in hair growth will be measured.

### Example VII

An 18 year old Middle Eastern female complaining of post chemo hair thinning and thinning eyebrows uses a specified pretreatment shampoo on her scalp and eyebrows (this shampoo allows compounds that follow shampoo treatment to penetrate the hair follicles to a greater degree). The patient sprays 0.3%/.01% w/v bimatoprost/cyclosporine solution to her hair and eyebrows at bedtime for 90 days. Hair growth is measured weekly by calculating the percent of affected area per cm2 demonstrating growth, as measured by AOI software, via Canfield photography. After 120 days of daily application of the 0.2%/0.4% w/v bimatoprost/cyclosporine A solution, a 31% increase in scalp growth and eyebrow growth will be measured.

### Example VIII

A 58 year old post menopausal female suffering from diffuse thinning hair throughout her scalp applies iontorphoresis patches at night on top of the areas of diffuse hair thinning (for a specified period of time) impregnated with a combination of 0.3%/0.5% w/v bimatoprost/cyclosporine solution. Hair growth is measured weekly by calculating the percent of affected area per cm2 demonstrating growth, as measured by AOI software, via Canfield photography. After 60 days of nightly application of the impregnated iontorphoresis patches 0.3%/0.5% w/v bimatoprost/cyclosporine A solution, a 41% increase in scalp growth will be measured.

### Example IX

A 25 year old Asian male suffering from diffuse hair loss uses a specially formulated conditioner designed to allow better follicular penetration of the bimatoprost/cyclosporine formulation followed by application of 0.3%/0.5% w/v bimatoprost/cyclosporine A emulsion to his hair once a day with a roll-on applicator for a period of 90 days. Hair growth is measured weekly by calculating the percent of affected area per cm2 demonstrating growth, as measured by AOI software, via Canfield photography. After 90 days of daily application of the 0.30/0/0.5% w/v bimatoprost/cyclosporine A emulsion a 38% increase in hair growth and fullness will be measured.

### Example X

A 34 year old Latin female, 6 weeks post partum suffers from patchy hair loss following the delivery of her twins. This patient shampoos and conditions her hair each night (the shampoo and conditioner that is being used allows the product/compound that follows the shampoo/conditioner combination to be absorbed to a greater degree). She than applies a gel that contains a variety penetrants as well as 0.2%/0.4% w/v bimatoprost/cyclosporine A to her hair once nightly for a period of 120 days. The gel is applied to the scalp and is allowed to dry. Hair growth is measured weekly by calculating the percent of affected area per cm2 demonstrating growth, as measured by AOI software, via Canfield photography. After 120 days of daily application of the 0.2%/0.4% w/v bimatoprost/cyclosporine A gel, a 36% increase in hair growth will be measured.

### Example XI

A 32 year old Caucasian male suffers from alopecia totalis applies a 0.1%/0.3% bimatoprost/cyclosporine A foam to his scalp once in the morning and once at night, for a period of 120 days. The foam is applied and allowed to remain on the entire scalp for 15 minutes after pretreatment of the skin with a warmed, vibratory device to increase the ability of the scalp to absorb compounds that follow this type of pretreatment regiment. After 90 days of application of the 0.1%/0.3% bimatoprost/cyclosporine A foam, sustained hair growth will be 1 noted to occur on the scalp. This is documented by using AOI software and Canfield photography. Additionally, hair growth is documented to take place at twice the rate in normal individuals that are not using the regiment noted above.

## Claims

1. A composition for growing hair in a patient wherein the composition comprises 0.03% w/v bimatoprost and 0.05% w/v cyclosporine A.

2. The composition of claim 1 wherein the composition further includes ethanol, propylene glycol, D-limonene and water.

3. The composition of claim 2 wherein the composition further includes at least one excipient selected from the group consisting of Transcutol®, Labrasol®, cineole, Cremophor RH-40, DMSO, oleic acid, isopropyl myristate, propylene glycol, oxybutynin and monolaurate.

4. The compositions of claim 3 wherein the composition is in the form of one selected from the group consisting of a liquid, suspension, emulsion, reverse emulsion, micro-emulsion, foam, semi-solid, solution, dispersion, capsule, gel, lotion, cream, paste, and polish.

5. The composition of claim 2 wherein the composition is selected from the group consisting of a solution, foam, emulsion and gel.

6. The composition of claim 5 wherein the composition is applied by an applicator.

7. The composition of claim 3 wherein the composition consists essentially of 0.03% bimatoprost, 0.05% cyclosporine A, ethanol, propylene glycol, D-limonene and water.

8. The composition of claim 7 wherein the composition further includes transcutol.

9. A composition comprising 0.03%) w/v bimatoprost and 0.05% w/v cyclosporine A, for use in a method of treating hair loss in a patient.

10. The composition of claim 9 wherein the method includes applying a foam of 0.03% w/v bimatoprost and 0.05% w/v cyclosporine A at least once a day to the scalp.

11. The composition of claim 10 wherein prior to application of the foam, the scalp is pretreated by application to the scalp of at least one of the following selected from the group consisting of heat, mechanical stimulation, sonophoresis, vibration and massage.

12. The composition of claim 11 wherein the composition is applied on the scalp by application of a roll-on applicator.

13. A non-therapeutic method of growing hair on a human scalp comprising application of a composition comprising 0.03% w/v bimatoprost and 0.05 % w/v cyclosporine A.

14. The non-therapeutic method of claim 13 wherein the composition is applied at least once a day.

15. The non-therapeutic method of claim 13 wherein the composition is applied as one selected from the group consisting of a shampoo and conditioner.

16. The non-therapeutic method of claim 13 wherein the composition is in the form of a liquid, suspension, emulsion, reverse emulsion, micro-emulsion, foam, semi-solid, solution, dispersion, capsule, gel, lotion, cream, paste, and polish.

## Patentansprüche

1. Zusammensetzung für das Haarwachstum bei einem Patienten, wobei die Zusammensetzung 0,03 % G/V Bimatoprost und 0,05 % G/V Cyclosporin A umfasst.

2. Zusammensetzung gemäss Anspruch 1, wobei die Zusammensetzung ferner Ethanol, Propylenglykol, D-Limonen und Wasser einschliesst.

3. Zusammensetzung gemäss Anspruch 2, wobei die Zusammensetzung ferner zumindest einen Hilfsstoff, ausgewählt aus der Gruppe bestehend aus Transcutol®, Labrasol®, Cineol, Cremophor RH-40, DMSO, Ölsäure, Isopropylmyristat, Propylenglykol, Oxybutinin und Monolaurat, einschliesst.

4. Zusammensetzung gemäss Anspruch 3, wobei die Zusammensetzung in Form von einem, ausgewählt aus der Gruppe bestehend aus einer/einem Flüssigkeit, Suspension, Emulsion, umgekehrten Emulsion, Mikroemulsion, Schaum, Halbfeststoff, Lösung, Dispersion, Kapsel, Gel, Lotion, Creme, Paste und Glanzmittel, vorliegt.

5. Zusammensetzung gemäss Anspruch 2, wobei die Zusammensetzung aus der Gruppe bestehend aus einer/einem Lösung, Schaum, Emulsion und Gel ausgewählt ist.

6. Zusammensetzung gemäss Anspruch 5, wobei die Zusammensetzung mit einem Applikator aufgetragen wird.

7. Zusammensetzung gemäss Anspruch 3, wobei die Zusammensetzung im wesentlichen aus 0,03 % Bimatoprost, 0,05 % Cyclosporin A, Ethanol, Propylenglykol, D-Limonen und Wasser besteht.

8. Zusammensetzung gemäss Anspruch 7, wobei die Zusammensetzung ferner Transcutol einschliesst.

9. Zusammensetzung, umfassend 0,03 % G/V Bimatoprost und 0,05 % G/V Cyclosporin A, zur Verwendung in einem Verfahren zur Behandlung von Haarausfall bei einem Patienten.

10. Zusammensetzung gemäss Anspruch 9, wobei das Verfahren das Auftragen eines Schaums aus 0,03 % G/V Bimatoprost und 0,05 % G/V Cyclosporin A zumindest einmal pro Tag auf die Kopfhaut einschliesst.

11. Zusammensetzung gemäss Anspruch 10, wobei die Kopfhaut vor dem Auftragen des Schaums durch Anwendung von zumindest einem der nachstehenden, ausgewählt aus der Gruppe bestehend aus Wärme, mechanischer Stimulierung, Sonophorese, Vibration und Massage, auf die Kopfhaut vorbehandelt wird.

12. Zusammensetzung gemäss Anspruch 11, wobei die Zusammensetzung durch Anwendung eines Roll-on-Applikators auf die Kopfhaut aufgetragen wird.

13. Nicht-therapeutisches Verfahren für Haarwachstum auf einer menschlichen Kopfhaut, das das Auftragen einer Zusammensetzung, umfassend 0,03 % G/V Bimatoprost und 0,05 % G/V Cyclosporin A, umfasst.

14. Nicht-therapeutisches Verfahren gemäss Anspruch 13, wobei die Zusammensetzung zumindest einmal pro Tag aufgetragen wird.

15. Nicht-therapeutisches Verfahren gemäss Anspruch 13, wobei die Zusammensetzung als eines, ausgewählt aus der Gruppe bestehend aus einem Shampoo und einem Konditionierer, aufgetragen wird.

16. Nicht-therapeutisches Verfahren gemäss Anspruch 13, wobei die Zusammensetzung in Form einer/eines Flüssigkeit, Suspension, Emulsion, umgekehrten Emulsion, Mikroemulsion, Schaums, Halbfeststoffs, Lösung, Dispersion, Kapsel, Gels, Lotion, Creme, Paste und Glanzmittels vorliegt.

## Revendications

1. Composition pour la croissance des cheveux chez un patient dans laquelle la composition comprend 0,03 % p/v de bimatoprost et 0,05 % p/v de cyclosporine A.

2. Composition selon la revendication 1 dans laquelle la composition inclut en outre de l'éthanol, du propylène glycol, du D-limonène et de l'eau.

3. Composition selon la revendication 2 dans laquelle la composition inclut en outre au moins un excipient sélectionné dans le groupe consistant en Transcutol®, Labrasol®, cinéole, Crémophor RH-40, DMSO, acide oléique, myristate d'isopropyle, propylène glycol, oxybutynine et monolaurate.

4. Composition selon la revendication 3 dans laquelle la composition se présente sous la forme de l'un sélectionné dans le groupe consistant en un liquide, une suspension, une émulsion, une émulsion inverse, une micro-émulsion, une mousse, un semi-solide, une solution, une dispersion, une capsule, un gel, une lotion, une crème, une pâte et un lustrant.

5. Composition selon la revendication 2 dans laquelle la composition est sélectionnée dans le groupe consistant en une solution, une mousse, une émulsion et un gel.

6. Composition selon la revendication 5 dans laquelle la composition est appliquée par un applicateur.

7. Composition selon la revendication 3 dans laquelle la composition consiste essentiellement en 0,03 % de bimatoprost, 0,05 % de cyclosporine A, éthanol, propylène glycol, D-limonène et eau.

8. Composition selon la revendication 7 dans laquelle la composition inclut en outre du transcutol.

9. Composition comprenant 0,03 % p/v de bimatoprost et 0,05 % p/v de cyclosporine A, pour son utilisation dans un procédé de traitement de la perte de cheveux chez un patient.

10. Composition selon la revendication 9 dans laquelle le procédé inclut l'application d'une mousse de 0,03 % p/v de bimatoprost et 0,05 % p/v de cyclosporine A au moins une fois par jour sur le cuir chevelu.

11. Composition selon la revendication 10 dans laquelle avant l'application de la mousse, le cuir chevelu est prétraité par l'application sur le cuir chevelu d'au moins l'un des suivants sélectionnés dans le groupe consistant en chaleur, stimulation mécanique, sonophorèse, vibrations et massage.

12. Composition selon la revendication 11 dans laquelle la composition est appliquée sur le cuir chevelu par application avec un applicateur à bille.

13. Procédé non thérapeutique pour la croissance des cheveux sur un cuir chevelu humain comprenant l'application d'une composition comprenant 0,03 % p/v de bimatoprost et 0,05 % p/v de cyclosporine A.

14. Procédé non thérapeutique selon la revendication 13 dans laquelle la composition est appliquée au moins une fois par jour.

15. Procédé non thérapeutique selon la revendication 13 dans lequel la composition est appliquée en tant que l'un sélectionné dans le groupe consistant en un shampooing et un conditionneur.

16. Procédé non thérapeutique selon la revendication 13 dans lequel la composition se présente sous la forme d'un liquide, d'une suspension, d'une émulsion, d'une émulsion inverse, d'une micro-émulsion, d'une mousse, d'un semi-solide, d'une solution, d'une dispersion, d'une capsule, d'un gel, d'une lotion, d'une crème, d'une pâte et d'un lustrant.
